(19) Europäisches Patentamt
European Patent Office
Office européen des brevets

(11) **EP 4 202 848 A1**

(12) **EUROPEAN PATENT APPLICATION**

(43) Date of publication:
**28.06.2023 Bulletin 2023/26**

(21) Application number: **21217713.3**

(22) Date of filing: **24.12.2021**

(51) International Patent Classification (IPC):
**G06T 19/20** (2011.01)     **G06T 5/00** (2006.01)
**G06T 7/10** (2017.01)

(52) Cooperative Patent Classification (CPC):
**G06T 19/20; G06T 5/005; G06T 7/12;**
G06T 2207/30004; G06T 2210/41; G06T 2219/2021

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **EWALD, Arne
Eindhoven (NL)**
• **WENZEL, Fabian
Eindhoven (NL)**
• **FLAESCHNER, Nick
Eindhoven (NL)**
• **HAUFE, Stefan
Eindhoven (NL)**

(74) Representative: **Philips Intellectual Property &
Standards
High Tech Campus 52
5656 AG Eindhoven (NL)**

(54) **MODEL BASED REPAIR OF THREE DIMENTIONAL TOMOGRAPHC MEDICAL IMAGING DATA**

(57)     Disclosed herein is a medical system (100, 300, 400) comprising: a memory (110) storing machine executable instructions (120) and a computational system (104). Execution of the machine executable instructions causes the computational system to: receive (200) modified three-dimensional tomographic medical image data (122) that has been facially erased within a facial zone (128); fit (202) a model-based segmentation (126) to the modified three-dimensional tomographic medical image data, wherein the model-based segmentation comprises multiple surface meshes (602, 604, 606, 608, 700) that define anatomical regions (800); label (204) each of the voxels of a reconstructed region (130) of the modified three-dimensional tomographic medical image data with an anatomical label according to anatomical regions defined by the multiple surface meshes; and assign (206) a contrast value to voxels in the reconstructed region using the anatomical label to provide repaired three-dimensional tomographic medical image data (132).

Fig. 7

**Description**

TECHNICAL FIELD

The invention relates to tomographic medical imaging data, in particular to the repair of three-dimensional tomographic medical image data.

BACKGROUND

Various tomographic medical imaging techniques such as Magnetic Resonance Imaging (MRI) and Computed Tomography enable detailed visualization of anatomical structure of a subject. A current trend is to modify the facial region of tomographic medical imaging data so that it cannot be identified before storage in a medical database or even in sets of data for training and test purposes. A drawback is that data which has been altered in this way will likely not function with much of the library of numerical algorithm for automatically processing and analyzing this data.

International patent application publication WO 2020/198560 A1 discloses that facial information in medical images may be de-identified using an approach that reduces undesired effects of de-identification on image statistics, while still protecting participant privacy. Rather than remove or blur face voxels, face voxels are replaced with image data from a template face, which in some instances may be a population-average face. Participant facial features are fully removed but impacts on downstream biomarker measurements are minimized by generating an output image that resembles a complete craniofacial image with statistical image texture properties similar to the original.

SUMMARY

The invention provides for a medical system, a computer program product, and a method in the independent claims. Embodiments are given in the dependent claims.

In WO 2020/198650, templated image data is used to replace portions of data that have been removed or obscured in a facial zone. This document only discloses or shows the repair of two-dimensional slices of data. A template as disclosed in this document would be unsuitable for repairing three-dimensional data sets. A template would likely not fit a three-dimensional data set properly.

Embodiments may provide a means for improving the quality of repaired three-dimensional tomographic medical image data by fitting a model-based segmentation to modified three-dimensional tomographic medical image data. The model-based segmentation comprises multiple surface meshes which three-dimensionally define volumes that correspond to anatomical regions. These models are able to fit arbitrarily shaped three-dimensional data sets. In addition, a model-based segmentation is able to flexibly adapt to variations and inconsistencies in the size and/or shape of a facial zone.

The model is then used to label voxels in the modified three-dimensional tomographic medical image data which are inside of a reconstructed region with a anatomical label. The reconstructed region comprises at least the facial zone. The anatomical label is then used to reconstruct facial structures within the facial zone resulting in repaired three-dimensional tomographic medical image data. Because the facial features in the repaired facial zone are defined by the surface meshes of the model-based segmentation they no longer resemble the subject from which tomographic medical image data was acquired. However, this repaired tomographic medical image data is able to be used in numerical algorithms and calculations which rely on facial structures being present.

In one aspect the invention provides for a medical system that comprises a memory that stores machine-executable instructions and a computational system. This embodiment of the medical system could be incorporated into different types of systems and used in different situations. In one example, the medical system is a cloud-based or remote-based server that may be used for performing image reconstruction and image processing tasks. In another embodiment the medical system is a computer system or workstation that is accessible to a radiologist or other healthcare professional. In yet other examples, the medical system may be incorporated or may comprise a medical imaging system, such as a CT or computed tomography system or a magnetic resonance imaging system or MRI system.

Execution of the machine-executable instructions causes the computational system to receive modified three-dimensional tomographic medical image data that is descriptive of a head of a subject. The modified three-dimensional tomographic medical image data comprises voxels. The three-dimensional tomographic medical image data has been facially erased within a facial zone. The term 'modified three-dimensional tomographic medical image data' is a label used to indicate or identify a particular three-dimensional tomographic medical image dataset or data. Facially erased, as used herein, encompasses having either facial characteristics that are removed or blurred. The effect of this is that it is impossible or more difficult to identify the subject from the modified three-dimensional tomographic medical image data. As used herein, the modified three-dimensional tomographic medical image data is descriptive of a head but incorporates a field of view that encompasses at least a brain or the subject.

Execution of the machine-executable instructions further causes the computational system to fit a model-based segmentation to the modified three-dimensional tomographic medical image data. The model-based segmentation model is a whole head. The model-based segmentation comprises multiple surface meshes that define anatomical regions. The whole head, as used herein, encompasses a region of a subject's head which incorporates at least the entire skull and surrounding soft tissues. The model-based segmentation defines these surface meshes that are used to divide the regions of the head into the different anatomical regions. For example, various bone and tissue structures may be identified by the surface meshes. In some embodiments the model-based segmentation may use a balance between internal and external energies to distort or modify the surface meshes. In the case where the facial zone has been facially erased with voxels either being removed or blurred, this portion of the face may be exempt from external constraints. For example, the model may be fit to the regions of the subject's head outside of the facial zone and then the model within the facial zone is allowed to simply snap and fit to match the rest of the model.

Execution of the machine-executable instructions further causes the computational system to label each of the voxels of a reconstructed region of the modified three-dimensional tomographic medical image data with an anatomical label according to anatomical regions defined by the multiple surface meshes. The reconstructed region comprises at least the facial zone. There are several different variants. In one case, the reconstructed region could be identical with the facial zone. In this case only those regions which have been erased or blurred are then labeled. In another case, the reconstructed region is larger than the facial zone but smaller than the entire head or field of view of the subject. This for example would mean that the areas which are labeled extend beyond the facial zone. In another example, the reconstructed region is identical with the subject's entire head or the field of view for the modified three-dimensional tomographic medical image data.

Execution of the machine-executable instructions further causes the computational system to assign a contrast value to voxels in the reconstructed region using the anatomical label to provide repaired three-dimensional tomographic medical image data. As is described below, there are several ways of assigning the contrast value or the value within a voxel. This embodiment may be beneficial because it enables a way of repairing the modified three-dimensional tomographic medical image data. There has been a current trend where tomographic medical image data has the facial zone facially erased or blurred. This causes a variety of technical problems. Well-known algorithms for segmenting the head or particularly the brain of a subject may rely on anatomical markers or structures that are within the facial zone to function properly. Another technical difficulty is that tomographic medical image data, which has been facially erased, may not be useful for training machine learning algorithms such as neural networks. The construction of the repaired three-dimensional tomographic medical image data enables this tomographic medical image data that had been facially erased, to be used in other algorithms and also to be used for training machine learning modules.

One potential advantage of using a model-based segmentation to fit the modified three-dimensional tomographic medical image data is that it may be useful in a variety of situations and provide for better replacement of voxels in the facial zone and/or the reconstructed region. For example, the modified three-dimensional tomographic medical image data could be a three-dimensional dataset, or it could even be a stack of two-dimensional datasets that are used to form a three-dimensional dataset. The model-based segmentation is able to adapt to these different layers or granularity of the three-dimensional data. This may for example provide better results than using such techniques as using a template to replace portions of the facial zone.

In another embodiment execution of the machine-executable instructions further causes the computational system to use the repaired three-dimensional tomographic medical image data in any one of the following: a segmentation algorithm, an electroencephalography algorithm, a magnetoencephalography algorithm, a transcranial magnetic stimulation planning algorithm, a transcranial direct current simulation planning algorithm, and a transcranial alternating current stimulation planning algorithm. The replacement of the voxels in the facial zone are particularly beneficial when using algorithms or planning algorithms that rely on electromagnetic calculations.

In another embodiment execution of the machine-executable instructions further causes the computational system to use the model-based segmentation as input to the following: the electroencephalography algorithm, the magnetoencephalography algorithm, the transcranial magnetic stimulation planning algorithm, the transcranial direct current simulation planning algorithm, and the transcranial alternating current stimulation planning algorithm for electromagnetic calculations within the head of the subject. This embodiment may be particularly beneficial because the segmentation defines the different regions of tissue types within the head of the subject. Typically, finite element techniques are used for doing electromagnetic calculations. With a knowledge of the segmentation this enables the electromagnetic calculations to be performed using a forward calculation that does not use finite elements. This may significantly reduce the calculation time needed to apply any one of these algorithms.

In another embodiment execution of the machine-executable instructions causes the computational system to assign the contrast value to voxels in the reconstructed region using a lookup table that comprises entries for each anatomical label. This embodiment may be beneficial because it provides for a very simple, effective and fast means of filling the voxels in the reconstructed region. This may be particularly useful when the reconstructed region is identical with the facial zone or say only slightly larger and does not encompass the brain region of the subject. When using a lookup table the resulting tomographic medical image that has been repaired may not look equivalent to a tomographic medical image that has not had the facial features removed and then repaired, however, it may be very effective in various types of numerical algorithms. In particular, it may function extremely well for segmentation algorithms, and it also may function extremely well for electromagnetic calculations that solve the electromagnetic field or equations using a finite elements method.

In another embodiment execution of the machine-executable instructions further causes the computational system to normalize the lookup table using regions of the modified three-dimensional tomographic medical image outside of the facial zone before assigning the contrast values to voxels in the reconstructed region. In various CT, MRI or other tomographic imaging methods the contrast from image-to-image may vary. Therefore, normalizing the lookup table or adjusting its values to match the modified three-dimensional tomographic medical image data may provide for a more natural and effective result.

In another embodiment execution of the machine-executable instructions further causes the computational system to perform smoothing and/or contrast matching at the boundary of the reconstructed region. This may be beneficial because there may be slight discontinuities at the boundary between the reconstructed region and portions of the modified three-dimensional tomographic medical image data that are outside of the reconstructed region. Adding a smoothing or contrast matching at the boundary may provide for a repaired three-dimensional tomographic medical image data that is more effective in numerical algorithms.

In another embodiment the memory further comprises a reconstruction neural network configured to assign the contrast value to the voxels in the reconstructed region in response to receiving the modified three-dimensional tomographic medical image data and the anatomical label of each voxel of the reconstructed region. As used herein, the term 'reconstruction neural network' is used to identify a particular neural network. The reconstruction neural network is a neural network. The reconstruction neural network could for example be formed from a pix2pix neural network or a CycleGAN neural network.

The reconstruction neural network may be configured to take two things as input. One is the label of the voxels within the reconstructed region as to their anatomical value, and the other is to take the modified three-dimensional tomographic medical image data as input. It is relatively straightforward to construct a neural network for doing this and for receiving both sets of data. It is fairly common to be able to expand various neural network architectures to receive color data that is data that may for example be formatted in RGB format where there are three numbers. In this case, neural networks can be extended in the same way except they are expanded to have two inputs. One input would be the contrast or value of voxels in the modified three-dimensional tomographic medical image data and the other would be to receive a value which encodes the anatomical region.

For example, a particular number or range of numbers could be used to encode different types of tissues or anatomical regions of the subject. The placement of the values of voxels in the reconstructed region could be done in different ways. In one example, the reconstruction neural network outputs the entire image with the voxels of the reconstructed region having their values replaced. This is similar to the idea of an autoencoder except the additional data in the anatomical data enables the neural network to replace all of the voxels. In other cases, the output of the neural network is received and then an additional algorithm takes the values of the voxels within the reconstructed region and then pastes them into the modified three-dimensional tomographic medical image data. In this way, voxels outside of the reconstructed region are not replaced by the reconstruction neural network.

The reconstruction neural network can be trained in a variety of different ways. In one example, one could take complete three-dimensional datasets of tomographic medical image data and prepare training data from it completely. In one instance, the original three-dimensional datasets are used as the ground truth data and the input for supervised or deep learning is taken by taking the same datasets and performing a facial blurring or removal of the facial zone of them. The original images, which have not been modified, can also then be segmented with the model-based segmentation. We then have the datasets with the facial region that is blurred or removed along with the segmentation, which can then be input into the neural network and then compared to the original dataset. Large numbers of these datasets can be prepared and can be used for supervised or deep learning.

There are also a variety of classic GAN approaches which will work. For example, a pix-to-pix scan would work well, where one translates a label mask into a realistic looking magnetic resonance image. Another example would be a cycle GAN which could be used, which uses unpaired images. There are literally a variety of ways in which GAN neural networks can be used.

In another embodiment the model-based segmentation comprises a defined brain volume. For example, the defined brain volume may be a volume which outlines a boundary of the subject's brain. Execution of the machine-executable instructions causes the computational system to ensure that the brain volume of the repaired three-dimensional tomographic medical image data is identical with the brain volume of the modified three-dimensional tomographic medical image data. In the case where the reconstructed region comprises all or a portion of the brain volume, then the computational system may take the values for the original voxels within the brain volume from the modified three-dimensional tomographic medical image data. In the case where the reconstructed region does not extend into the brain volume, then there would be no need to do this.

In another embodiment the modified three-dimensional tomographic medical image data is a three-dimensional dataset or a stack of two-dimensional slices.

In another embodiment the modified three-dimensional tomographic medical image data is magnetic resonance imaging data or it is computed tomography imaging data.

In another embodiment the model-based segmentation is a deformable shape model segmentation.

In another embodiment the model-based segmentation is a vocally deformable statistical shape model segmentation.

In another embodiment the model-based segmentation is an active contour model segmentation.

In another embodiment execution of the machine-executable instructions further causes the computational system to receive acquired three-dimensional tomographic medical image data. Execution of the machine-executable instructions further causes the computational system to receive the modified three-dimensional tomographic medical image data in response to inputting the acquired three-dimensional tomographic medical image data into a facial removal algorithm. A facial removal algorithm, as used herein, encompasses an algorithm which may determine a facial zone using a segmentation or registration technique. The facial removal algorithm may take voxels within this facial region and either set them to a predetermined value such as zero or may blur them with their neighbors to obscure facial features.

In another embodiment the medical system further comprises a tomographic medical imaging system. Execution of the machine-executable instructions further causes the computational system to control the tomographic medical imaging system to acquire the acquired three-dimensional tomographic medical imaging data.

In another aspect the invention provides for a method of medical imaging. The method comprises receiving modified three-dimensional tomographic medical imaging data descriptive of a head of a subject and comprising voxels. The three-dimensional tomographic medical imaging data has been facially erased within a facial zone. The method further comprises fitting a model-based segmentation to the modified three-dimensional tomographic medical image data. The model-based segmentation model is a whole head. The model-based segmentation comprises multiple surface meshes that define anatomical regions.

The method further comprises labeling each of the voxels of a reconstructed region of the modified three-dimensional tomographic medical image data with an anatomical label according to anatomical regions defined by the multiple surface meshes. The reconstructed region comprises at least the facial zone. The method further comprises assigning a contrast value to voxels in the reconstructed region using the anatomical label to provide repaired three-dimensional tomographic medical imaging data.

In another aspect the invention provides for a computer program product that comprises machine-executable instructions for execution by a computational system. For example, they may be machine-executable instructions on a non-transitory storage medium. Execution of the machine-executable instructions causes the computational system to receive modified three-dimensional tomographic medical image data descriptive of a head of a subject and comprising voxels. The three-dimensional tomographic medical imaging data has been facially erased within a facial zone. Execution of the machine-executable instructions further causes the computational system to fit a model-based segmentation to the modified three-dimensional tomographic medical image data. The model-based segmentation model is a whole head. The model-based segmentation comprises multiple surface meshes that define anatomical regions.

Execution of the machine-executable instructions further causes the computational system to label each of the voxels of a reconstructed region of the modified three-dimensional tomographic medical image data with an anatomical label according to anatomical regions defined by the multiple surface meshes. These are regions which are defined by being between particular multiple surface meshes. The reconstructed region comprises at least the facial zone. Execution of the machine-executable instructions further causes the computational system to assign a contrast value to voxels in the reconstructed region using the anatomical label to provide repaired three-dimensional tomographic medical image data.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Three-dimensional tomographic medical image data is defined herein as being the reconstructed three-dimensional visualization of anatomic data as imaged by a tomographic medical imaging system such as a computed tomographic system or a magnetic resonance imaging system.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer. A three-dimensional magnetic resonance image or a stack of two-dimensional magnetic resonance images is an example of three-dimensional tomographic medical image data.

Computed tomography measurement data is defined as being the measurements acquired by a Computed Tomography system when imaging a subject. For example, the computed tomographic data may be the acquire X-ray attenuation profiles.

A computed tomography (CT) image is the reconstructed two- or three-dimensional visualization of anatomic data contained within the computed tomography measurement data. A three-dimensional computed tomography image or a stack of two-dimensional computed tomography images is an example of three-dimensional tomographic medical image data.

BRIEF DESCRIPTION OF THE DRAWINGS

In the following preferred embodiments of the invention will be described, by way of example only, and with reference to the drawings in which:

Fig. 1 illustrates an example of a medical system;
Fig. 2 shows a flow chart which illustrates a method of using the medical system of Fig. 1;
Fig. 3 illustrates a further example of a medical system;
Fig. 4 illustrates a further example of a medical system;

Fig. 5 depicts a two-dimensional slice of exemplary modified three-dimensional tomographic medical image data;
Fig. 6 illustrates an example of a shape model formed from multiple surface meshes;
Fig. 7 shows the adaption of the shape model of Fig. 6 to the magnetic resonance image 122 of Fig. 5; and
Fig. 8 shows a two-dimensional cross section of the shape model of Figs. 6 and 7.

DESCRIPTION OF EMBODIMENTS

Like numbered elements in these figures are either equivalent elements or perform the same function. Elements which have been discussed previously will not necessarily be discussed in later figures if the function is equivalent.

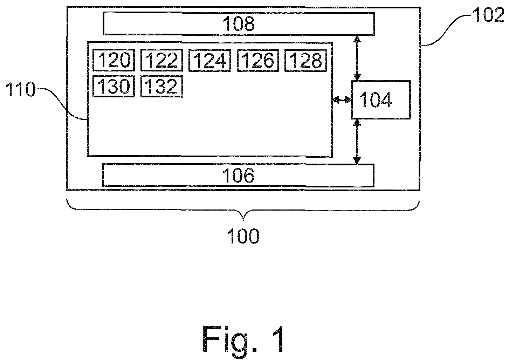

Fig. 1 illustrates an example of a medical system 100. The medical system 100 is shown as comprising a computer 102 with a computational system 104. The computer 102 is intended to represent one or more computers or computing devices located at one or more locations. Likewise, the computational system 104 could include one or more processing or computational cores and could be located at a single location or could be distributed.

The medical system 100 is shown as further comprising an optional user interface 106. The optional user interface 106 would for example enable the operator of the medical system 100 to interact with it and/or control the medical system. The medical system 100 is shown as comprising an optional network connection 108. The network connection 108 may for example be used by the medical system 100 to communicate with other systems and, for example, the medical system 100 as depicted could be located at a remote location across the internet and assessed by various centers where tomographic medical imaging is performed. The medical system 100 is further shown as containing a memory 110 that is accessible to the computational system 104. The memory 110 is intended to represent various types of memory and storage devices which are accessible to the computational system 104.

The memory 110 is shown as storing machine-executable instructions 120. The machine-executable instructions 120 enable the computational system 104 to perform various tasks such as controlling other components if they are present, as well as performing various image processing and numerical tasks such as image reconstruction or the image processing of tomographic medical imaging data. The memory 110 is further shown as containing modified three-dimensional tomographic medical image data 122. The modified three-dimensional tomographic medical image data 122 has been facially erased within a facial zone. This means that the voxels which are in a facial region of the subject have either been set to a predetermined value or have been blurred or smeared such that anatomical details are no longer intelligible. The memory 110 is further shown as containing a segmentation module 124. The segmentation module 124 may be executable code which enables the fitting of a model-based segmentation 126 to the modified three-dimensional tomographic medical image data 122.

The segmentation module 124 may in some examples be modified such that fitting parameters in the facial zone do not use anatomical landmarks or constraints to match to the modified three-dimensional tomographic medical image data 122. For example, when using a shape deformable model the other portions of the modified three-dimensional tomographic medical image data 122 may be used and the model-based segmentation 126 simply snaps to fit the rest of the model. The memory 110 is further shown as containing an optional identification of the location of a facial zone 128. For example, an algorithm could be used to detect the location of voxels where the facial features of the subject have been erased. In other examples, the identification of the location of the facial zone 128 could be provided as metadata that accompanies the modified three-dimensional tomographic medical image data 122.

For example, if the facial features in the facial zone were erased by an automatic algorithm, this could have appended the modified three-dimensional tomographic medical image data with a description of which voxels have been erased or modified. The memory 110 further contains an identification of a location of a reconstructed region 130. This is the region where voxels have anatomical labels assigned to them. As was mentioned previously, this could be exclusively the facial zone, it could be the entire region of interest, which contains the subject's head, or it could be an intermediate value. In some instances, the identification of the location of the reconstructed region 130 may be provided by an automatic algorithm or it may be received from the user interface 106. The memory 110 is further shown as containing repaired three-dimensional tomographic medical image data 132 that was prepared by replacing voxels in the reconstructed region 130 using anatomical labels assigned to the voxels in the reconstructed region. As will be described below, this may be achieved in several different ways.

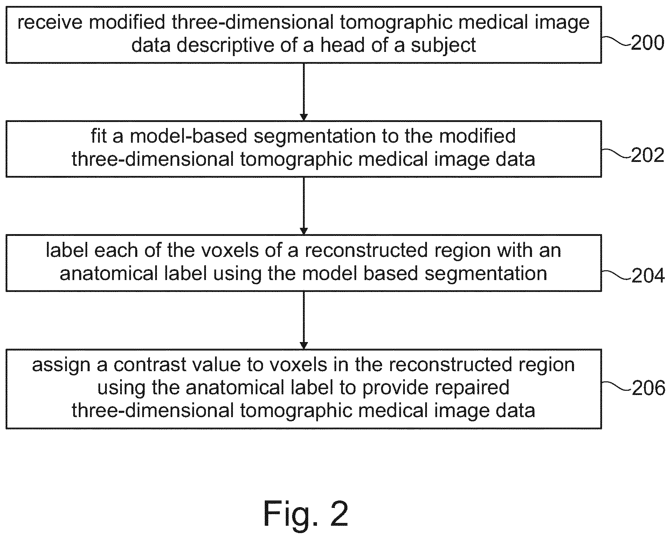

Fig. 2 shows a flowchart which illustrates a method of operating the medical system of Fig. 1. First, in step 200, the modified three-dimensional tomographic medical image data 122 is received. The modified three-dimensional tomographic medical image data 122 is descriptive of a head of a subject. The modified three-dimensional tomographic medical image data 122 comprises voxels and there is a facial zone where the medical image data has been facially erased. This means that a portion of the three-dimensional tomographic medical image data 122 is descriptive of a facial region of the subject and this region has either been set to a predetermined value or blurred so as to obscure facial features. Next, in step 202, a model-based segmentation 126 is fit to the modified three-dimensional tomographic medical image data 122. The model-based segmentation 126 comprises multiple surface meshes that define anatomical regions. For example, these surface meshes define boundaries between different anatomical regions. The regions between different surface meshes therefore have a particular anatomical region or tissue type.

Next, in step 204, each of the voxels of a reconstructed region 130 are labeled with an anatomical label that is according to the anatomical regions defined by the multiple surface meshes. The reconstructed region comprises at least the facial zone. Finally, in step 206, a contrast value is assigned to voxels in the reconstructed region 130 using the anatomical label to provide repaired three-dimensional tomographic medical image data. This could be achieved in a variety of different ways. In one case, only the voxels in the facial zone 128 are replaced. In another example, all of the voxels are replaced. In this case the voxels which are descriptive of the brain of the subject may then be later pasted into the new image to provide the repaired three-dimensional tomographic medical image data. In other cases, the reconstructed region could be an intermediate region that includes a transitional zone between the facial zone and the rest of the modified three-dimensional tomographic medical image data.

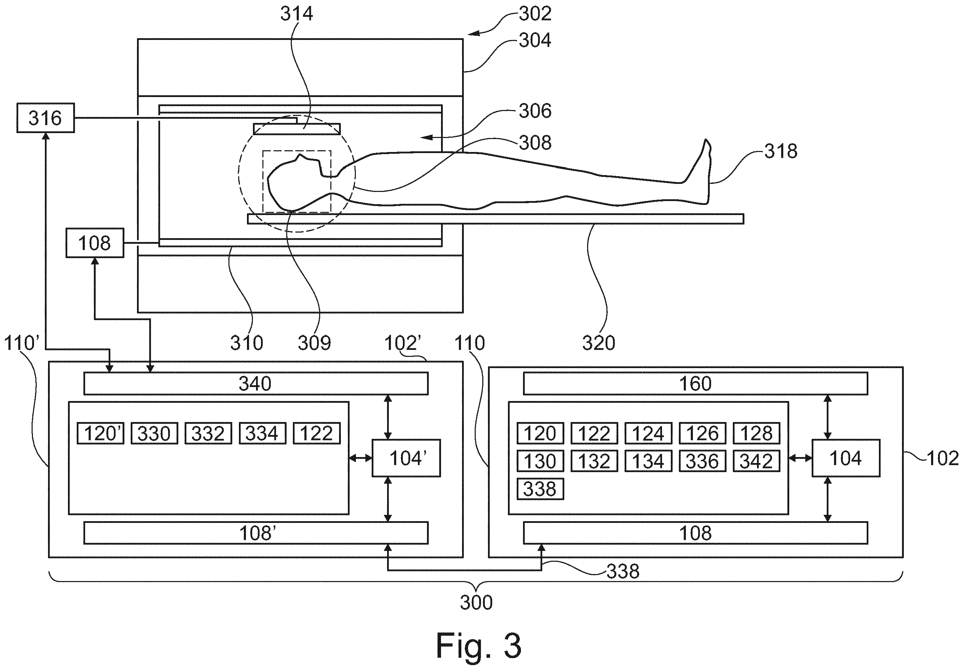

Fig. 3 illustrates a further example of the medical system 300. The medical system 300 is similar to the medical system 100 depicted in Fig. 1 except it additionally comprises a magnetic resonance imaging system 302 that has a controller 102'.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data that is acquired typically acquried for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for acquisition of preliminary k-space data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels.

The transceiver 316 and the gradient controller 312 are shown as being connected to the hardware interface 106 of the computer system 102.

The controller 102' is similar to the computer 102. It comprises a computational system 104' and a network interface 108'. Additionally, there is a hardware interface 340, which enables the computational system 104' to control the operation and function of the magnetic resonance imaging system 302. The controller 102' further comprises a memory 110', which is also representative of the various types of memory and storage accessible to the computational system 104'. In some examples, the features of the controller 102' and the computer 102 could be combined. However, it is likely that the computer 102 would be a separate system that accesses the modified three-dimensional tomographic medical image data 122 from a database or storage system.

The memory 110' is shown as comprising machine-executable instructions 120' which comprise instructions that enable the computational system 104' to control the magnetic resonance imaging system 302 using the hardware interface 340. This comprises such things as sending commands for the control as well as receiving data acquired by the magnetic resonance imaging system 302.

The memory 110' is further shown as containing pulse sequence commands 330 that may be used by the computational system 104' to control the magnetic resonance imaging system 302 to acquire k-space data 332 descriptive of the field of view 309. The memory 110' is shown as further containing the three-dimensional magnetic resonance image data 334 that has been reconstructed from the k-space data 332. The k-space data 332 was acquired by controlling the magnetic resonance imaging system 302 with the pulse sequence commands 330. The memory 110' is further shown as containing the modified three-dimensional tomographic medical image data 122. In this example, the modified three-dimensional tomographic medical image data 122 was constructed from the three-dimensional magnetic resonance imaging data 334 by facially erasing voxels in the facial zone. This may have been performed by an operator manually or it may have been performed by an automatic algorithm, which could be for example programmed into the machine-executable instructions 120'.

A network connection 338 between the network interface 108 and 108' enables the computer 102 and the controller 102' to exchange data. In this case the modified three-dimensional tomographic medical image data 122 was sent from the controller 102' to the computer 102. The computer 102, depicted in Fig. 3, is similar to the computer 102 depicted in Fig. 1 except the memory 110 is further shown as containing a reconstruction neural network 336. The reconstruction neural network 336 takes the mapping of the anatomical regions and at least the reconstruction region 134 and the modified three-dimensional tomographic medical image data 122 as input. In response, it outputs a generated three-dimensional magnetic resonance image data 342.

The generated three-dimensional magnetic resonance image data 342 is essentially a synthetic image that the reconstruction neural network 336 generates. Voxels from the generated three-dimensional magnetic resonance image data 342 and the modified three-dimensional tomographic medical image data 122 can be used to construct the repaired three-dimensional tomographic medical image data 132. For example, voxels within the reconstructed region 130 can be pasted into the modified three-dimensional tomographic medical image data 122. As an alternative, voxels from just the generated three-dimensional magnetic resonance image data 342 into the modified three-dimensional tomographic medical image data 122. There are a variety of different ways in which the modified three-dimensional tomographic medical image data 122 could be reconstructed. In another example, the generated three-dimensional magnetic resonance image data 342 is used in its entirety and then just the voxels which encompass a brain volume from the modified three-dimensional tomographic medical image data 122 are pasted into the generated three-dimensional magnetic resonance image data 342 to make the repaired three-dimensional tomographic medical image data 132. In this example, the anatomical landmarks and such of the generated three-dimensional magnetic resonance image data 342 are used and then the data descriptive of the subject's brain from the original image is used.

The memory 110 is further shown as containing a mapping of anatomical regions in the at least reconstructed region 134. This mapping is an assignment of anatomical regions to the various voxels within the reconstructed region 130. The model-based segmentation 126 divides the modified three-dimensional tomographic medical image data 122 into different anatomical regions. It is then very straightforward to assign a value to each one of these.

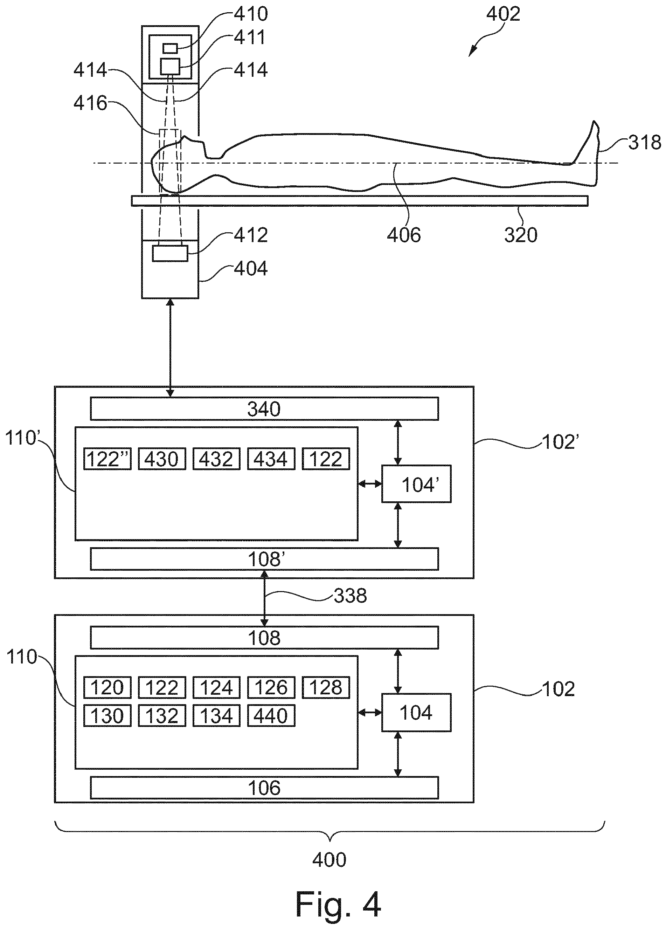

Fig. 4 illustrates a further example of a medical system 400. The medical system 400 in Fig. 4 is similar to the medical system 300 in Fig. 3 except instead of comprising a magnetic resonance imaging system 302; it instead comprises a computed tomography system 402.

The CT system 402 comprises a rotating gantry 404. The gantry 404 rotates about an axis of rotation 406. There is a subject 318 reposing on a subject support 320. Within the gantry 404 is the X-ray tube 410.

The X-ray tube 410 produces X-rays 414 that pass through a collimator 411 and then through the subject 318 and are received by a detector 412. Within the area of the box 416 is an imaging zone where CT or computer tomography images of the subject 418 can be acquired. The CT system 402 is shown as being controlled by the controller 102'. The hardware interface 304 allows the processor 106 to exchange messages and control the CT system 502.

In this example, the contents of the memory 110' of the controller 102' are different. In this example, there are machine-executable instructions 120" that again provide for image processing tasks that also enable the computational system 104' to control the computed tomography system 402 via the hardware interface 340. The memory 110' further comprises CT system control commands 430. These are specifically the commands that the computational system 104' uses to control the computed tomography system 402 to acquire computed tomography measurement data 432. The machine-executable instructions 120" also enable the computational system 104' to reconstruct three-dimensional computed tomography data 434 from the computed tomography measurement data 432. And again, the machine-executable instructions 120" cause the computational system 104' to construct the modified three-dimensional tomographic medical image data 122 from the three-dimensional computed tomography data 434. The controller 102' may send the modified three-dimensional tomographic medical image data 122 via the network connection 338 to the computer 102.

In the example in Fig. 3, a reconstruction neural network 336 was used. In this example, a lookup table 440 is used instead. The lookup table 440 takes the mapping of anatomical regions in at least the reconstructed region 134 and then assigns intensities or contrasts to each of these voxels using the lookup table 440. In some examples, the values of the lookup table 440 may be adjusted by examining regions within the modified three-dimensional tomographic medical image data 122 to match contrasts. After the substitution of voxels within the reconstructed region 130, the modified three-dimensional tomographic medical image data 122 is transformed into the repaired three-dimensional tomographic medical image data 132.

In some clinical applications, facial parts are necessary for subsequent algorithmic processing of the image, e.g. for volume conductor generation in electrophysiological applications such as EEG/MEG or TMS/TDCS/TACS. Furthermore, also segmentation algorithms might benefit from facial context information.

In current applications, the facial area is just removed, resulting in a non-realistic shape. This may cause severe problems, e.g. for volume conductor generation, due to modelling inaccuracies and the important role of hard tissues such as bone. This especially relates to finite element modelling (FEM) which is usually more precise compared to boundary element modelling (BEM).

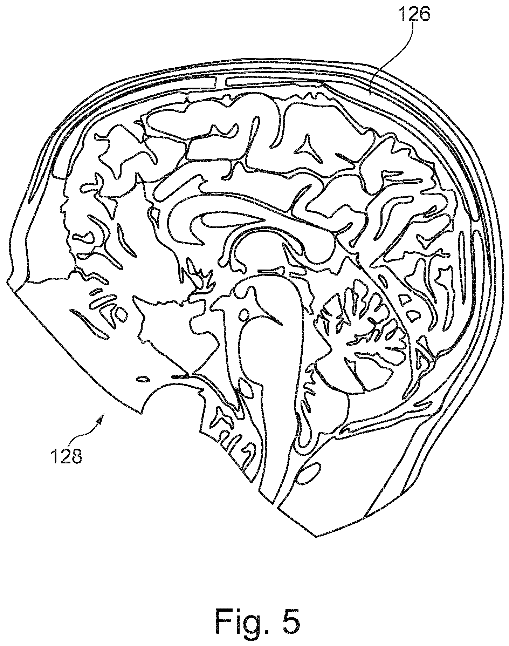

Fig. 5 illustrates a single slice of modified three-dimensional tomographic medical image data 122. In this example, the modified three-dimensional tomographic medical image data 122 is a magnetic resonance image. It can be clearly seen that there is a facial zone 128 where the facial features have been removed by setting them to the same value as the background.

Examples may provide for a tool that substitutes a patient's face that has been removed in an MRI or other tomographic scan. This can be achieved by a series of processing steps: a) optionally removing the facial parts with a classical de-identification algorithm, b) applying model-based segmentation (MBS) to the new image in order to generate a smooth representation of the individual anatomy and the generic face c) generating an image from the MBS surface mesh c) blending the generic facial part into the image where the patient face has been removed.

A triangular surface model may be constructed containing anatomical structures such as skin, skull, air and brain hemispheres. This surface model is trained to adapt to unseen MR images.

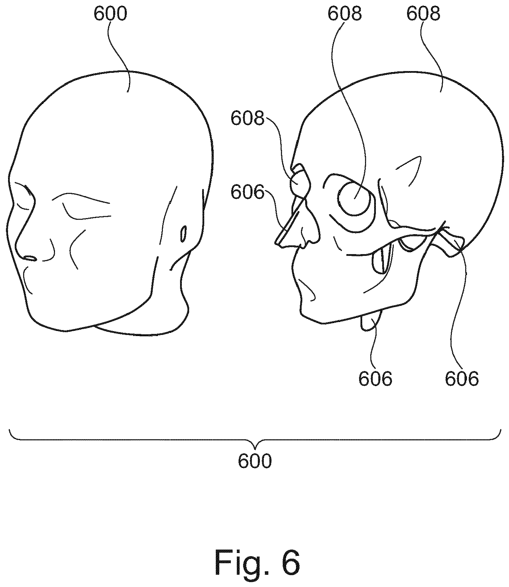

Fig. 6 illustrates an example of a shape model 600 with several surface meshes visible. The region labeled 602 is a surface mesh which defines the boundary of the skin. The regions labeled 606 represent surfaces which define airways 606 or air cavities. The circular regions labeled 608 define the boundary of the eyes 608 of the subject.

During adaptation, each mesh triangle looks for a target point in the image space along its normal. The target points are characteristic image features (typical grey values, edges) learnt beforehand during model construction. The target points found by the triangles comprise the so-called external energy termed $E_{ext}$. Triangles distant to image features do not search for target points and, hence, do not contribute to the external energy. Hence, the external energy is driven by parts of the image containing image features whereas the de-faced regions are neglected for computing the external energy. It can be expressed by

$$E_{ext} = \sum_{i \in \{T_p\}} w_i \left( \frac{\tilde{n}_i}{\|\tilde{n}_i\|} (x_i - c_i) \right)^2 \tag{1}$$

with $\{T_p\}$ being the set of triangles, $\tilde{n}_i = (\tilde{n}_1 \ \tilde{n}_2 \ \tilde{n}_3)^T$ the normal vector at the target point $x_i$ and $c_i$ the triangle center of the i-th triangle. Additionally, the weights $w_i$ are large for reliable target points and small otherwise. As a slight modification to Equation (1), one could also sum over all mesh triangles and set the weights for triangles outside part of the image volume containing image features to zero.

In contrast to the external energy, the internal energy Eint does not consider the image information or image features at all. It is responsible for a) penalizing deviations between the current state of the mesh and the mesh from the personalized 3D model and b) for ensuring shape consistency. It is given by

$$E_{int} = \sum_{i=1}^{V} \sum_{j \in \{N(i)\}} \left( (v_i - v_j) - (m_i - m_j) \right)^2, \tag{2}$$

where V denotes the number of vertices and $\{N(i)\}$ the set of neighboring vertices of vertex i with coordinates $v_i = (v_1 \ v_2 \ v_3)^T$ in the current mesh. The vector $m_i$ denotes the vertex coordinates of the i-th vertex in the reference mesh obtained from the initial 3D segmentation. One can observe from Equation (2) that the internal energy only depends on the current mesh and the reference 3D mesh.

The final deformation is achieved by minimizing the sum of the internal and external energy:

$$E := \alpha E_{int} + (1 - \alpha) E_{ext}, \tag{3}$$

with $\alpha$ being a parameter to balance the influence of each energy. Different parts of the mesh can contribute with different internal energy weights or stiffnesses. For this invention, the facial areas would obtain a rather large value.

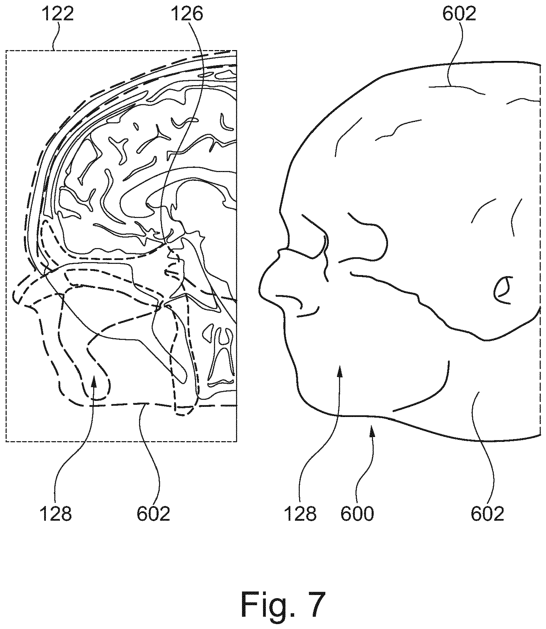

Fig. 7 shows the adaption of the shape model of Fig. 6 to the magnetic resonance image 122 of Fig. 5. Please note that this is just for illustrative purposes and the model is not optimized for this application which leads to non-smooth mesh boundaries. Fig. 7 shows a further view of the magnetic resonance image that was depicted in Fig. 5. In this case the model-based segmentation 126 has been fit to the magnetic resonance image 122. It can be seen that various elements of the model-based segmentation 126 extend into the facial zone 128. A surface mesh representing the skin 602 is shown as on the exterior and also in a view to the right of the magnetic resonance image 122.

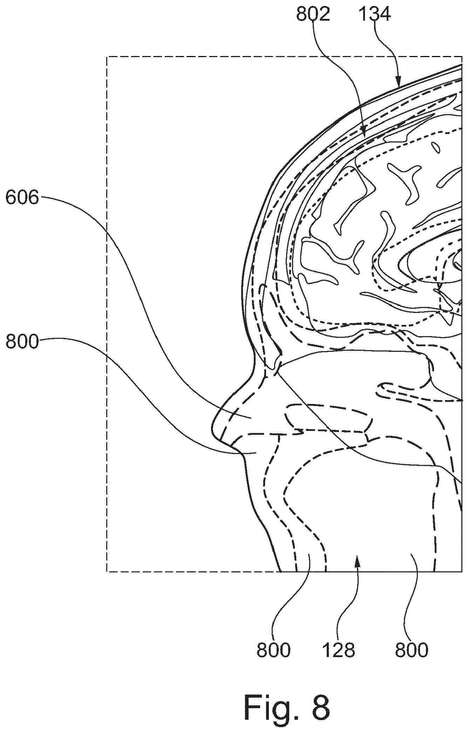

After the model-based segmentation, the surface mesh needs to be transformed to voxel-space. This is achieved by labelling each voxel within a specific region as a member of this region. Fig. 8 shows an example. Finally, a machine learning algorithm for segmentation, image classification or an FEM algorithm can exploit the segmentation map with incorporates a generic face.

Fig. 8 illustrates an example of a segmentation map 134 (or mapping of anatomical regions) that incorporates a number of surface meshes 700 that define distinct anatomical regions 802. This can be used with a lookup table to repair the modified three-dimensional tomographic medical image data 122 or it may also be used as input to the reconstruction neural network 336. Which for example may be realized as a so-called Generative-Adversarial Network (GAN). Here, an artificial neural network is trained to map a de-faced image to an image containing a face.

Also visible in the segmentation 134 is a contour representing the boundary of a brain volume 802. In some examples the voxels from the brain volume 802 of modified three-dimensional tomographic medical image data are pasted or copied into the corresponding brain volume 802 of the repaired three-dimensional tomographic medical image data 132. This may ensure that during clinical examination the voxels in the brain volume 802 are the subject's and not synthetically generated.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

REFERENCE SIGNS LIST

| | |
|---|---|
| 100 | medical system |
| 102 | computer |
| 104 | computational system |
| 106 | optional user interface |
| 108 | network connection |
| 110 | memory |
| 120 | machine executable instructions |
| 122 | modified three-dimensional tomographic medical image data |
| 124 | segmentation module |
| 126 | model-based segmentation |
| 128 | identification of location of facial zone |
| 130 | identification of location of reconstructed region |
| 132 | repaired three-dimensional tomographic medical image data |
| 134 | mapping of anatomical regions in at least the reconstructed region |
| 200 | receive modified three-dimensional tomographic medical image data descriptive of a head of a subject and comprising voxels |
| 202 | fit a model-based segmentation to the modified three-dimensional tomographic medical image data, wherein the model-based segmentation models a whole head |
| 204 | label each of the voxels of a reconstructed region of the modified three-dimensional tomographic medical image data with an anatomical label according to anatomical regions defined by the multiple surface meshes |
| 206 | assign a contrast value to voxels in the reconstructed region using the anatomical label to provide repaired three-dimensional tomographic medical image data |

| | |
|---|---|
| 300 | medical system |
| 302 | magnetic resonance imaging system |
| 304 | magnet |
| 306 | bore of magnet |
| 308 | imaging zone |
| 309 | field of view |
| 310 | magnetic field gradient coils |
| 312 | magnetic field gradient coil power supply |
| 314 | radio-frequency coil |
| 316 | transceiver |
| 318 | subject |
| 320 | subject support |
| 330 | pulse sequence commands |
| 332 | k-space data |
| 334 | three-dimensional magnetic resonance image data |
| 336 | reconstruction neural network |
| 338 | network connection |
| 340 | hardware interface |
| 342 | generated three-dimensional magnetic resonance image data |
| 400 | medical system |
| 402 | CT system |
| 404 | gantry |
| 406 | axis of rotation |
| 410 | X-ray tube |
| 411 | collimator |
| 412 | detector |
| 414 | X-rays |
| 416 | imaging zone |
| 420 | subject support acutator |
| 430 | CT system control commands |
| 432 | computed tompgraphy measurement data |
| 434 | three-dimensional computed tomography data |
| 440 | look-up table |
| 600 | shape model |
| 602 | skin |
| 604 | skull |
| 606 | airways |
| 608 | eyes |
| 700 | surface meshes |
| 800 | distinct anatomical regions |
| 802 | brain volume |

**Claims**

1. A medical system (100, 300, 400) comprising:

   - a memory (110) storing machine executable instructions (120); and
   - a computational system (104), wherein execution of the machine executable instructions causes the computational system to:

      - receive (200) modified three-dimensional tomographic medical image data (122) descriptive of a head of a subject (318) and comprising voxels, wherein the modified three-dimensional tomographic medical image data has been facially erased within a facial zone (128);
      - fit (202) a model-based segmentation (126) to the modified three-dimensional tomographic medical image data, wherein the model-based segmentation models a whole head, wherein the model-based segmentation comprises multiple surface meshes (602, 604, 606, 608, 700) that define anatomical regions (800);
      - label (204) each of the voxels of a reconstructed region (130) of the modified three-dimensional tomographic medical image data with an anatomical label according to anatomical regions defined by the multiple surface meshes, wherein the reconstructed region comprises at least the facial zone; and
- assign (206) a contrast value to voxels in the reconstructed region using the anatomical label to provide repaired three-dimensional tomographic medical image data (132).

2. The medical system of claim 1, wherein execution of the machine executable instructions further causes the computational system to use the repaired three-dimensional tomographic medical image data in any one of the following: a segmentation algorithm, an electroencephalography algorithm, a magnetoencephalography algorithm, a transcranial magnetic stimulation planning algorithm, a transcranial direct current stimulation planning algorithm, and a transcranial alternating current stimulation planning algorithm.

3. The medical system of claim 2, wherein execution of the machine executable instructions further causes the computational system to use the model-based segmentation as input to any one of the following: the electroencephalography algorithm, the magnetoencephalography algorithm, the transcranial magnetic stimulation planning algorithm, the transcranial direct current stimulation planning algorithm, and the transcranial alternating current stimulation planning algorithm for electromagnetic calculations within the head of the subject.

4. The medical system of claim 1, 2, or 3, wherein execution of the machine executable instructions causes the computational system to assign the contrast value to voxels in the reconstructed region using a lookup table that comprises entries for each anatomical label.

5. The medical system of claim 4, wherein execution of the machine executable instructions causes the computational system to normalize the lookup table using regions of the modified three-dimensional tomographic medical image outside of the facial zone before assigning the contrast value to voxels in the reconstructed region.

6. The medical system of claim 5, wherein execution of the machine executable instructions further causes the computational system to perform smoothing and/or contrast matching at a boundary of the reconstructed region.

7. The medical system of claims 1, 2, or 3, wherein the memory further comprises a reconstruction neural network configured to assign the contrast value to the voxels in the reconstructed region in response to receiving the modified three-dimensional tomographic medical image data and the anatomical label of each voxel of the reconstructed region.

8. The medical system of any one of the preceding claims, wherein the model-based segmentation comprises a brain volume, wherein execution of the machine executable instructions causes the computational system ensure that the brain volume of the repaired three-dimensional tomographic medical image data is identical with the brain volume of the modified three-dimensional tomographic medical image data.

9. The medical system of any one of the preceding claims, wherein the modified three-dimensional tomographic medical image data is a three-dimensional data set or a stack of two-dimensional slices.

10. The medical system of any one of the preceding claims, wherein the modified three-dimensional tomographic medical image data is any one of the following: magnetic resonance imaging data and computed tomography imaging data.

11. The medical system of any one of the preceding claims, wherein the model-based segmentation is any one of the following: a deformable shape model segmentation, an active contour model, and a locally deformable statistical shape model segmentation.

12. The medical system of any one of the preceding claims, wherein execution of the machine executable instructions further causes the computational system to:

- receive acquired three-dimensional tomographic medical image data (334, 434);
- receive the modified three-dimensional tomographic medical image data in response to inputting the acquired three-dimensional tomographic medical image data into a facial removal algorithm.

13. The medical system of any one of the preceding claims, wherein the medical system further comprises a tomographic medical imaging system (302, 402), wherein execution of the machine executable instructions further causes the computational system to control the tomographic medical imaging system to acquire the acquired three-dimensional tomographic medical image data.

14. A method of medical imaging, wherein the method comprises:

- receiving (200) modified three-dimensional tomographic medical image data (122) descriptive of a head of a subject (318) and comprising voxels, wherein modified the three-dimensional tomographic medical image data has been facially erased within a facial zone (128);
- fitting (202) a model-based segmentation (126) to the modified three-dimensional tomographic medical image data, wherein the model-based segmentation models a whole head, wherein the model-based segmentation comprises multiple surface meshes (602, 604, 606, 608, 700) that define anatomical regions;
- labeling (204) each of the voxels of a reconstructed region (130) of the modified three-dimensional tomographic medical image data with an anatomical label according to anatomical regions defined by the multiple surface meshes, wherein the reconstructed region comprises at least the facial zone; and
- assigning (206) a contrast value to voxels in the reconstructed region using the anatomical label to provide repaired three-dimensional tomographic medical image data (132).

15. A computer program product comprising machine executable instructions for execution by a computational system (104), wherein execution of the machine executable instructions causes the computational system to:

- receive (200) modified three-dimensional tomographic medical image data (122) descriptive of a head of a subject (318) and comprising voxels, wherein the modified three-dimensional tomographic medical image data has been facially erased within a facial zone (128);
- fit (202) a model-based segmentation to the modified three-dimensional tomographic medical image data, wherein the model-based segmentation models a whole head, wherein the model-based segmentation comprises multiple surface meshes (602, 604, 606, 608, 700) that define anatomical regions (800);
- label (204) each of the voxels of a reconstructed region (130) of the modified three-dimensional tomographic medical image data with an anatomical label according to anatomical regions defined by the multiple surface meshes, wherein the reconstructed region comprises at least the facial zone; and
- assign (206) a contrast value to voxels in the reconstructed region using the anatomical label to provide repaired three-dimensional tomographic medical image data (132).

Fig. 1

```
┌─────────────────────────────────────────────────────┐
│  receive modified three-dimensional tomographic      │
│  medical image data descriptive of a head of a       │──200
│  subject                                             │
└─────────────────────────────────────────────────────┘
                          │
                          ▼
┌─────────────────────────────────────────────────────┐
│  fit a model-based segmentation to the modified      │
│  three-dimensional tomographic medical image data    │──202
└─────────────────────────────────────────────────────┘
                          │
                          ▼
┌─────────────────────────────────────────────────────┐
│  label each of the voxels of a reconstructed region  │
│  with an anatomical label using the model based      │──204
│  segmentation                                        │
└─────────────────────────────────────────────────────┘
                          │
                          ▼
┌─────────────────────────────────────────────────────┐
│  assign a contrast value to voxels in the            │
│  reconstructed region using the anatomical label     │
│  to provide repaired three-dimensional tomographic   │──206
│  medical image data                                  │
└─────────────────────────────────────────────────────┘
```

# Fig. 2

Fig. 3

Fig. 4

Fig. 5

Fig. 6

Fig. 7

Fig. 8

Europäisches
Patentamt

European
Patent Office

Office européen
des brevets

# EUROPEAN SEARCH REPORT

Application Number

EP 21 21 7713

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/378607 A1 (CHEN WEITIAN [CN] ET AL) 12 December 2019 (2019-12-12)<br>* figures 13-17 *<br>* paragraphs [0008], [0014], [0032] *<br>* paragraph [0035] *<br>* paragraph [0037] – paragraph [0074] * | 1-15 | INV.<br>G06T19/20<br>G06T5/00<br>G06T7/10 |
| A,D | WO 2020/198560 A1 (MAYO FOUND MEDICAL EDUCATION & RES [US])<br>1 October 2020 (2020-10-01)<br>* the whole document * | 1-15 | |
| A | MIKULAN EZEQUIEL ET AL: "A comparative study between state-of-the-art MRI deidentification and AnonyMI, a new method combining re-identification risk reduction and geometrical preservation",<br>HUMAN BRAIN MAPPING,<br>vol. 42, no. 17,<br>14 September 2021 (2021-09-14), pages 5523-5534, XP055927485,<br>ISSN: 1065-9471, DOI: 10.1002/hbm.25639<br>Retrieved from the Internet:<br>URL:https://onlinelibrary.wiley.com/doi/full-xml/10.1002/hbm.25639><br>* the whole document * | 1-15 | |
| A | MIKHAIL MILCHENKO ET AL: "Obscuring Surface Anatomy in Volumetric Imaging Data",<br>NEUROINFORMATICS, SPRINGER-VERLAG, NEW YORK,<br>vol. 11, no. 1,<br>12 September 2012 (2012-09-12), pages 65-75, XP035160080,<br>ISSN: 1559-0089, DOI: 10.1007/S12021-012-9160-3<br>* the whole document * | 1-15 | |

TECHNICAL FIELDS
SEARCHED (IPC)

G06T

-/--

The present search report has been drawn up for all claims

| Place of search | Date of completion of the search | Examiner |
|---|---|---|
| Berlin | 8 June 2022 | Höller, Helmut |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 21 21 7713

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 3 633 613 A1 (KONINKLIJKE PHILIPS NV [NL]) 8 April 2020 (2020-04-08) * figures 3, 4, 6 * * paragraphs [0002], [0006], [0008] * * paragraph [0009] * ----- | 1,14,15 | |
| A | HARGREAVES MITCHELL ET AL: "A Generative Deep Learning Approach for Forensic Facial Reconstruction", 2021 DIGITAL IMAGE COMPUTING: TECHNIQUES AND APPLICATIONS (DICTA), IEEE, 29 November 2021 (2021-11-29), pages 1-7, XP034058463, DOI: 10.1109/DICTA52665.2021.9647290 [retrieved on 2021-12-10] * figures 2, 3 * * chapter: III. "Experiment" * ----- | 1,14,15 | |
| A | CELONG LIU ET AL: "Superimposition-guided Facial Reconstruction from Skull", ARXIV.ORG, CORNELL UNIVERSITY LIBRARY, 201 OLIN LIBRARY CORNELL UNIVERSITY ITHACA, NY 14853, 29 September 2018 (2018-09-29), XP081088540, * the whole document * ----- | 1,14,15 | TECHNICAL FIELDS SEARCHED (IPC) |
| A | US 2012/230566 A1 (DEAN HOWARD D [US] ET AL) 13 September 2012 (2012-09-13) * figures 1, 1A, 21A, 21B, 34A-36B, 43A-45 * * paragraph [0008] - paragraph [0018] * * paragraph [0134] - paragraph [0225] * * paragraph [0292] - paragraph [0297] * ----- | 1,14,15 | |

The present search report has been drawn up for all claims

| Place of search | Date of completion of the search | Examiner |
|---|---|---|
| Berlin | 8 June 2022 | Höller, Helmut |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 7713

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2022

| Patent document cited in search report | | Publication date | Patent family member(s) | | Publication date |
|---|---|---|---|---|---|
| US 2019378607 | A1 | 12-12-2019 | NONE | | |
| WO 2020198560 | A1 | 01-10-2020 | NONE | | |
| EP 3633613 | A1 | 08-04-2020 | EP | 3633613 A1 | 08-04-2020 |
| | | | EP | 3861527 A1 | 11-08-2021 |
| | | | US | 2021350544 A1 | 11-11-2021 |
| | | | WO | 2020070024 A1 | 09-04-2020 |
| US 2012230566 | A1 | 13-09-2012 | US | 2012230566 A1 | 13-09-2012 |
| | | | US | 2015032421 A1 | 29-01-2015 |
| | | | US | 2016203241 A1 | 14-07-2016 |
| | | | US | 2018032641 A1 | 01-02-2018 |
| | | | US | 2018189421 A1 | 05-07-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020198560 A1

- WO 2020198650 A